# EUROPEAN PATENT APPLICATION

(11) **EP 4 480 386 A1**
(43) Date of publication of application: **25.12.2024**
(21) Application number: 22928120.9
(22) Date of filing: 04.07.2022
(51) Int. Cl.: A61B 1/05

(54) **3D ELECTRONIC ENDOSCOPE AND CAMERA SYSTEM THEREFOR**

(30) Priority: 24.02.2022 CN 202220402386 U
(71) Applicant: Wuhan Mindray Bio-Medical Scientific Co., Ltd., Wuhan City Hubei 430206 (CN); Shenzhen Mindray Bio-Medical Electronics Co., Ltd., Shenzhen, Guangdong 518057 (CN)
(72) Inventor: LI, Yang, Shenzhen, Guangdong 518057 (CN); YUAN, Xiaowen, Shenzhen, Guangdong 518057 (CN); WU, Xiaohua, Shenzhen, Guangdong 518057 (CN)
(74) Representative: KIPA AB
(86) International application number: PCT/CN2022/103722
(87) International publication number: WO 2023/159851

(57) **Abstract**

Provided is a 3D electronic endoscope, comprising an insertion portion (30) and an operation portion (40). The insertion portion (30) comprises a long tube (32), a camera module (31), an illuminating optical path and a transmission unit (70); the operation portion (40) comprises an operating handle housing, a controller and an image signal processing assembly (41). The controller and the image signal processing assembly (41) are arranged in the operating handle housing; the camera module (31) comprises a first optical path assembly, a second optical path assembly, a first sensor (314a), a second sensor (314b), a first flexible board (315a), a second flexible board (315b), and a fixed substrate (316); the first optical path assembly comprises a first objective lens group (311a) and a first prism group (312a), and the second optical path assembly comprises a second objective lens group (311b) and a second prism group (312b); the first optical path assembly and the second optical path assembly reflect or transmit the image light to the first sensor (314a) and the second sensor (314b); the first sensor (314a) and the second sensor (314b) are electrically connected to the first flexible board (315a) and the second flexible board (315b), respectively; the first sensor (314a) and the second sensor (314b) are arranged on two sides of the fixed substrate (316), the first sensor (314a) and the first flexible board (315a) are arranged on the same surface of the fixed substrate (316), and the second sensor (314b) and the second flexible board (315b) are arranged on the other surface of the fixed substrate (316).

## Description

### TECHNICAL FIELD

The disclosure relates to a field of an endoscope, and in particular to a 3D electronic endoscope and an imaging system thereof.

### BACKGROUND

A 3D electronic endoscope, such as a 3D laparoscope, generally adopts a technology of "chip in the tip", that is, an imaging module is placed at an end of an insertion portion, which end is away from an operation portion and is also called a distal end. Due to a limitation for a maximum width of the insertion portion of the 3D electronic endoscope, which width is generally required to be controlled within 10.5mm, a size of the imaging module cannot be too large. In order to obtain high-resolution images and high-quality outputs, it is hoped to use a sensor with a larger photosensitive surface. However, the sensor is an important component of the imaging module. Therefore, it is crucial to make full use of the limited space at the end of the insertion portion to maximize the effective photosensitive surface of the sensor.

### SUMMARY

In view of this, embodiments of this disclosure expect to provide an imaging head for a 3D electronic endoscope, which head has an improved image quality.

An embodiment of this disclosure provides an imaging head for 3D electronic endoscope, including an insertion portion and an operation portion; wherein the insertion portion includes a long tube, an imaging module, an illumination optical path and a transmission unit; the long tube is a tubular structure with a hollow interior, the imaging module is arranged inside a distal end of the long tube, wherein the distal end is an end which is away from the operation portion; the illumination optical path and the transmission unit are arranged inside the long tube; the illumination optical path is capable of connecting a light guide beam and is configured to illuminate a designated region of an inspected object with a light which is transmitted by a light source; the imaging module is configured to receive an image light which is reflected or excited by the designated region and obtained by a distal end of the insertion portion, convert the image light into an electrical signal, and transmit the electrical signal to the operation portion through the transmission unit;
wherein the operation portion includes an operation handle housing, a controller, and a processing component for image signal; the controller and the processing component for image signal are arranged inside the operation handle housing; the controller is configured to implement different functions according to signal(s) which is(are) inputted by an operator; the processing component for image signal is configured to process the electrical signal which is transmitted by the transmission unit, so as to obtain an image signal;
wherein the imaging module includes a first optical path component, a second optical path component, a first sensor, a second sensor, a first flexible board, a second flexible board, and a fixation substrate; the first optical path component includes a first objective lens group and a first prism group, the second optical path component includes a second objective lens group and a second prism group; the first objective lens group is configured to receive the image light along a first path which is reflected or excited by the designated region, the first prism group is configured to reflect or transmit the image light along the first path to the first sensor; the first sensor is electrically connected with the first flexible board, and configured to convert the image light along the first path into a first electrical signal; the second objective lens group is configured to receive the image light along a second path which is reflected or excited by the designated region; the second prism group is configured to reflect or transmit the image light along the second path to the second sensor; the second sensor is electrically connected with the second flexible board, and configured to convert the image light along the second path into a second electrical signal;
the first sensor and the second sensor are respectively arranged on respective sides of the fixation substrate; the first sensor and the first flexible board are arranged on one surface of the fixation substrate; the second sensor and the second flexible board are arranged on the other surface of the fixation substrate.

In an embodiment, the first sensor, the first flexible board, the fixation substrate, the second flexible board and the second sensor are stacked.

In an embodiment, the first flexible board is provided with a through-hole structure, wherein the first sensor is arranged inside said through-hole structure; and/or the second flexible board is provided with a through-hole structure, wherein the second sensor is arranged inside said through-hole structure.

In an embodiment, the first prism group includes right-angle prism(s) or triangular prism(s); and/or the second prism group includes right-angle prism(s) or triangular prism(s).

In an embodiment, a light-exiting end surface of the first prism group is a rectangle, a long side of said rectangle is parallel to an axial direction of the long tube, and a short side of said rectangle is perpendicular to the axial direction of the long tube; and/or a light-exiting end surface of the second prism group is a rectangle, a long side of said rectangle is parallel to an axial direction of the long tube, and a short side of said rectangle is perpendicular to the axial direction of the long tube.

In an embodiment, diaphragms is(are) further included, wherein the diaphragm(s) is(are) arranged on the light-exiting end surface of the first prism group and/or the light-exiting end surface of the second prism group.

In an embodiment, a photosensitive area on a photosensitive surface of the first sensor is greater than or equal to a light-exiting end surface of the first prism group; and/or a photosensitive area on a photosensitive surface of the second sensor is greater than or equal to a light-exiting end surface of the second prism group.

In an embodiment, the light-exiting end surface of the first prism group is arranged on the photosensitive surface of the first sensor, and at least part of the photosensitive area on the photosensitive surface of the first sensor is in a closed space; and/or the light-exiting end surface of the second prism group is arranged on the photosensitive surface of the second sensor, and at least part of the photosensitive area on the photosensitive surface of the second sensor is in a closed space.

In an embodiment, a glass plate is further included; wherein the glass plate is arranged between the first prism group and the first sensor, and/or the glass plate is arranged between the second prism group and the second sensor.

In an embodiment, the transmission unit includes a signal amplification circuit which is configured to amplify the first electrical signal and/or the second electrical signal obtained, and transmit the amplified signal(s) to the processing component for image signal.

In an embodiment, the fixation substrate includes a first fixation substrate and a second fixation substrate; wherein the first sensor and the first flexible board are arranged on the first fixation substrate; the second sensor and the second flexible board are arranged on the second fixation substrate.

In an embodiment, the fixation substrate is a heat conduction plate for conducting heat which is generated by the sensor.

In an embodiment, the fixation substrate is a ceramic plate or a metal plate.

In an embodiment, a support is further included, wherein the first optical path component and the second optical path component are arranged at the support.

An embodiment provides an imaging head for 3D electronic endoscope, including an insertion portion and an operation portion; wherein the insertion portion includes a long tube, an imaging module, an illumination optical path and a transmission unit; the long tube is a tubular structure with a hollow interior, the imaging module is arranged inside a distal end of the long tube, wherein the distal end is an end which is away from the operation portion; the illumination optical path and the transmission unit are arranged inside the long tube; the illumination optical path is capable of connecting a light guide beam and is configured to illuminate a designated region of an inspected object with a light which is transmitted by a light source; the imaging module is configured to receive an image light which is reflected or excited by the designated region and obtained by a distal end of the insertion portion, convert the image light into an electrical signal, and transmit the electrical signal to the operation portion through the transmission unit;
wherein the operation portion includes an operation handle housing, a controller, and a processing component for image signal; the controller and the processing component for image signal are arranged inside the operation handle housing; the controller is configured to implement different functions according to signal(s) which is(are) inputted by an operator; the processing component for image signal is configured to process the electrical signal which is transmitted by the transmission unit, so as to obtain an image signal;
wherein the imaging module includes an objective lens group, a prism group, a sensor, a fixation substrate, and a flexible board; wherein the objective lens group is configured to receive an image light which is reflected or excited by the designated region; the prism group is configured to reflect or transmit the image light which is received by the objective lens group to the sensor; wherein the sensor is electrically connected with the flexible board, and configured to convert the image light into an electrical signal; the flexible board is electrically connected with the transmission unit, and configured to transmit the electrical signal to the transmission unit;
the sensor and the flexible board are electrically connected with each other, wherein the sensor and the flexible board are arranged on the fixation substrate.

An embodiment provides an imaging system for 3D electronic endoscope, including a light source, a light guide beam, an imaging host, a cable, and a 3D electronic endoscope according to any of above embodiments; wherein the light source is connected with the 3D electronic endoscope through the light guide beam, and one end of the 3D electronic endoscope is connected with the imaging host through the cable.

### BRIEF DESCRIPTION OF THE DRAWINGS

FIG. 1 is a structural diagram of an imaging system for 3D electronic endoscope in an embodiment.
FIG. 2 is a structural diagram of an imaging system for 3D electronic endoscope in an embodiment.
FIG. 3 is a structural diagram of a sensor in an embodiment.
FIG. 4 is a structural diagram of an imaging module of a 3D electronic endoscope in an embodiment.
FIG. 5 is a structural diagram of an imaging module of a 3D electronic endoscope in an embodiment.

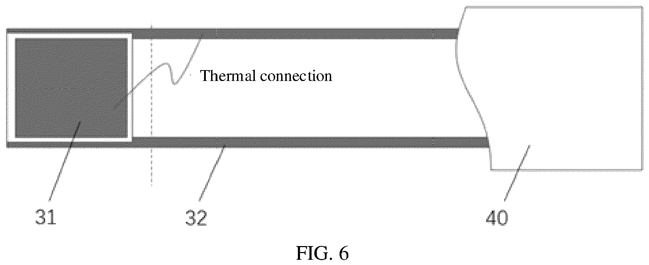

FIG. 6 is a structural diagram of a 3D electronic endoscope in an embodiment.

In the drawings: 10-light source, 20-light guide beam, 30-insertion portion, 31-imaging module, 311-objective lens group, 311a-first objective lens group, 311b-second objective lens group, 312-prism group, 312a-first prism group, 312b-second prism group, 313-support, 314a-first sensor, 314b-second sensor, 3141-photosensitive area, 3142-second welding portion, 315a-first flexible board, 315b-second flexible board, 316-fixation substrate, 32-long tube, 33-signal amplification circuit, 40-operation portion, 41-processing component for image signal, 50-imaging host, 60-display, 70-transmission unit, 71-cable, 72-video connection cable, 100-inspected object, 1000-imaging system for 3D electronic endoscope.

### DESCRIPTION OF THE PREFERRED EMBODIMENTS

Embodiments of this disclosure are further described in detail below in conjunction with the accompanying drawings and examples. The following embodiments are used to illustrate this disclosure but are not intended to limit the scope of this disclosure.

In the description of the embodiments of this disclosure, it should be noted that the terms "centre", "longitudinal", "lateral", "up", "down", "front", "back", "left", "right", "vertical", "horizontal", "top", "bottom", "inside", "outside", etc., indicate orientations or positional relationships based on the orientations or positional relationships shown in the accompanying drawings, and are only for facilitating describing the embodiments of this disclosure and simplifying the description, and do not indicate or imply that the referred device or components must have a specific orientation, be constructed and operated in a specific orientation, and therefore should not be understood as a limitation on the embodiments of this disclosure. Furthermore, terms "first", "second", and "third" are used for descriptive purposes only, and should not be understood as indicating or implying relative importance. "Distal end" described in embodiments of this disclosure refers to a portion of an insertion portion 30 of a long tube, which portion is away from an operation portion 40, and "proximal end" described in embodiments of this disclosure refers to a portion of an insertion portion 30 of a long tube, which portion is adjacent to the operation portion 40.

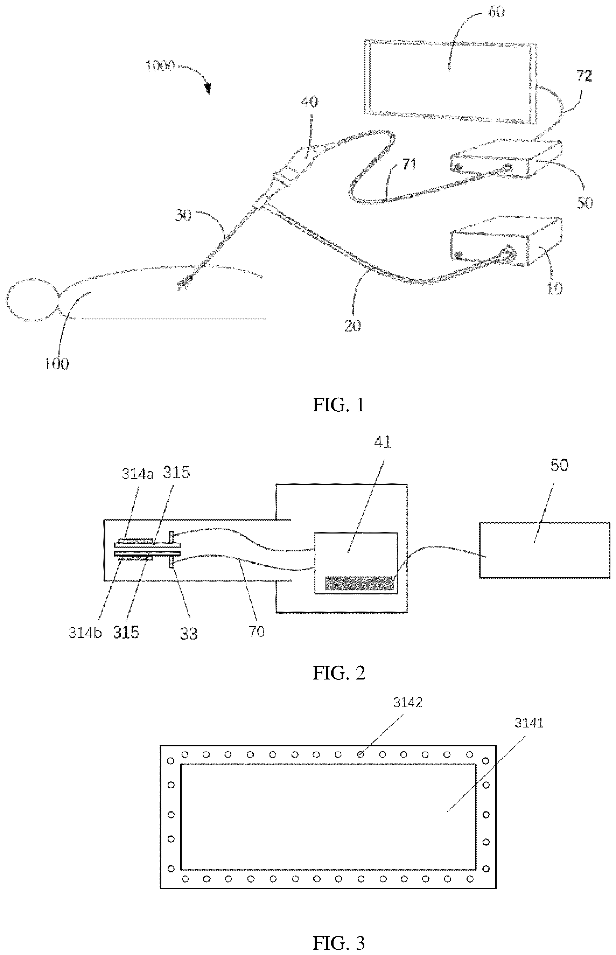

As shown in FIG. 1 and FIG. 2, in an embodiment, an imaging system for 3D electronic endoscope 1000 is provided, including a light source 10, a light guide beam 20, an electronic scope, a cable 71, an imaging host 50, a display 60 and a video connection cable 72.

The electronic scope is an important component of the imaging system for 3D electronic endoscope 1000, and includes an insertion portion 30 and an operation portion 40. The insertion portion 30 and the operation portion 40 may be an integrated structure. The insertion portion 30 includes a long tube 32, an imaging module 31, an illumination optical path and a transmission unit 70. The long tube 32 can be a circular long tube with a relatively smooth outer surface and a hollow interior. The long tube 32 is inserted into a body of an inspected object 100, so that an outer diameter of the long tube 32 needs to be as small as possible, such as ≤ 10.5 mm. In an embodiment, the long tube 32 can be made into a non-circular tube, such as an elliptical tube. The imaging module 31 is arranged inside a portion of the long tube 32, which portion is away from the operation portion 40, that is, the imaging module 31 is arranged at a distal end of the long tube 32, so as to receive an image light, which is reflected or excited by a designated region of the inspected object 100, convert the image light into an electrical signal, and transmit the electrical signal to the operation portion 40 through the transmission unit 70. Specifically, the imaging module 31 of the 3D electronic endoscope has two optical paths, which are equipped with a first optical path component and a second optical path component. The first optical path component receives an image light from a left or right side, and the second optical path component receives an image light from the other side. The left image light and the right image light are respectively transmitted to two sensors 314 (that is, a first sensor 314a, and a second sensor 314b). In order to ensure a final three-dimensional imaging effect, two sensors of a same model are preferably used. The first sensor 314a and the second sensor 314b respectively convert the image light along a first path and the image light along a second path obtained into a first electrical signal and a second electrical signal respectively, and transmit the first electrical signal and the second electrical signal to the operation portion 40 through the transmission unit 70, respectively. The illumination optical path is capable of being connected with the light guide beam 20, and is configured to illuminate the designated region of the inspected object 100 with a light which is transmitted by the light source 10. The transmission unit 70 is connected with the operation portion 40, and is configured to transmit the electrical signals which are outputted by the sensors 314 to the operation portion 40. The operation portion 40 includes an operation handle housing, a controller, and a processing component for image signal 41. The controller is configured to implement different functions according to signal(s) which is(are) inputted by an operator. For example, the controller can implement functions, such as a mode conversion function, a focus function, a color correction function, a lighting switch function, etc. The processing component for image signal 41 is configured to process the first electrical signal and the second electrical signal, which are transmitted by the transmission unit 70, so as to obtain a first image signal and a second image signal, and transmit the first image signal and the second image signal to the imaging host 50. The processing component for image signal can perform relay processing, serial-to-parallel conversion, differential transmission processing, etc., on the received electrical signals. In another embodiment, at least some electrical signal(s) of the first image signal and/or the second image signal may be converted by at least one image signal conversion unit into non-electrical signal(s), such as optical signal(s) from electrical signal(s).

The display 60 displays an image of the designated region of the inspected object 100. After the left and right image lights obtained by the 3D electronic endoscope are processed (such as polarization-processed), a corresponding image for the designated region of the inspected object 100 is displayed on the display 60. The observer needs to wear polarized glasses for observation, and finally observes a three-dimensional stereoscopic image.

A signal amplification circuit 33 may also be provided inside the 3D electronic endoscope. The signal amplification circuit 33 is provided between the imaging module 31 and the processing component for image signal 41, and is configured to amplify the electrical signal which is outputted by the imaging module 31, so as to ensure that the electrical signal is not excessively attenuated during transmission, thereby affecting the final imaging effect.

The imaging host 50 is connected with the 3D electronic endoscope via a cable 71, and the image signal generated by the 3D electronic endoscope is transmitted to the imaging host 50 via the cable 71 for processing. In some embodiments, the cable 71 may be an optical communication cable, such as an optical fibre. The 3D electronic endoscope converts the image signal (electrical signal) into an optical signal, which is transmitted to the imaging host 50 via the cable 71. The imaging host 50 then converts the optical signal into the electrical signal (image signal). In another embodiment, the cable 71 may also be a photoelectric composite cable. The 3D electronic endoscope converts some electrical signals into optical signals, retains some electrical signals, and transmits these signals to the imaging host 50 through the cable 71 for processing. In another embodiment, the 3D electronic endoscope can also transmit the image signal to the imaging host 50 for processing via wireless transmission.

Those skilled in the art should understand that FIG. 1 is merely an example of an imaging system for 3D electronic endoscope and does not constitute a limitation of the imaging system for 3D electronic endoscope. The imaging system for 3D electronic endoscope may include more or fewer units than shown in FIG.1, or combine certain units, or include different units.

In an embodiment, the light source 10 is configured to provide illumination or excitation light source to a region to be observed. The light source includes a white light source (illumination light source). In another embodiment, the light source 10 may also include an excitation light source (laser light source, such as near-infrared light) corresponding to a fluorescent agent.

In an embodiment, a processor is provided inside the imaging host 50, and the imaging host 50 is connected with the electronic scope via a cable 71 for obtaining an image signal which is outputted by the electronic scope. After the processor obtains the image signal which is outputted by the electronic scope, it processes the image signal to output a white light image or a fluorescent image of an observed tissue. The acquired image signal may be a separate white light signal or a fluorescent signal, or may be an image signal that is a combination of a white light signal and a fluorescent signal.

An embodiment of this disclosure provides an imaging head for 3D electronic endoscope, which imaging head can be used in the imaging system for 3D electronic endoscope 1000 provided in any one of above embodiments.

The 3D electronic endoscope includes an operation portion 40 and an insertion portion 30; wherein the insertion portion 30 and the operation portion 40 may be an integrated structure, and together constitute an imaging head for medical endoscope. Wherein the insertion portion 30 includes a long tube 32, an imaging module 31, an illumination optical path and a transmission unit 70. The long tube 32 is a tubular structure with a hollow interior, and the imaging module 31 is arranged inside the long tube 32. The illumination optical path and the transmission unit 70 are arranged inside the long tube 32; the illumination optical path is configured to illuminate a designated region of an inspected object 100 with a light from a light source 10. In an embodiment, the light source 10 and the 3D electronic endoscope are connected via a light guide beam 20. In another embodiment, the light source 10 is arranged at a distal end of the 3D electronic endoscope.

The imaging module 31 is configured to receive an image light which is reflected or excited by the designated region and obtained by a distal end of the insertion portion 30, convert the image light into an electrical signal, and transmit the electrical signal to the operation portion 40 through the transmission unit 70.

The operation portion 40 includes an operation handle housing, a controller and a processing component for image signal 41; the controller is configured to implement different functions according to signal(s), which are/is inputted by an operator; the processing component for image signal is configured to process the electrical signal which is transmitted by the transmission unit 70, so as to obtain an image signal. In another embodiment, the operation portion 40 may further include an image signal conversion unit for converting the image signal into another non-electrical signal.

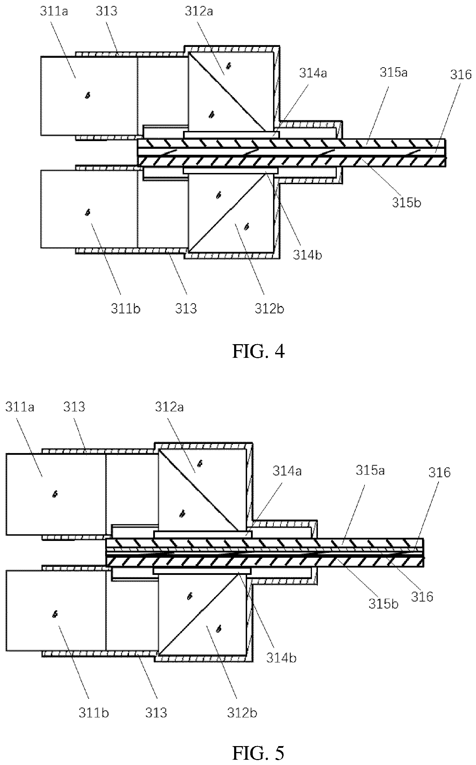

As shown in FIG. 4, the imaging module 31 includes a first optical path component, a second optical path component, a first sensor 314 a, a second sensor 314 b, a first flexible board 315 a, a second flexible board 315 b and a fixation substrate 316. The first flexible board 315 a and the second flexible board 315 b function as circuit boards to transmit the electrical signal to the transmission unit 70. As shown in FIG. 5, in an embodiment, the fixation substrate 316 may also be composed of multiple pieces which are joined together to form a whole. In another embodiment, the fixation substrate 316 may also be a ceramic plate or a metal plate. Ceramic plate has mature manufacturing processes and is moderately priced, so is a conventional material for making medical endoscopes. Metal plate can facilitate heat conduction and guide heat from the sensor 314 to the long tube.

The first optical path component includes a first objective lens group 311a and a first prism group 312a, and the second optical path component includes a second objective lens group 311b and a second prism group 312b. The first objective lens group 311a is configured to receive the image light along a first path which is reflected or excited by a designated region; the first prism group 312a is configured to reflect or transmit the image light along the first path to the first sensor 314a; the first sensor 314a is electrically connected with the first flexible board 315a, and configured to convert the image light along the first path into a first electrical signal. The second objective lens group 311b is configured to receive the image light along a second path which is reflected or excited by the designated region; the second prism group 312b is configured to reflect or transmit the image light along the second path to the second sensor 314b; the second sensor 314b is electrically connected with the second flexible board 315b, and configured to convert the image light along the second path into a second electrical signal.

The first flexible board 315a and the second flexible board 315b are electrically connected with the transmission unit 70, so as to transmit the first electrical signal and the second electrical signal to the transmission unit 70; the first sensor 314a and the second sensor 314b are respectively arranged on respective sides of the fixation substrate 316, wherein the first sensor 314a and the first flexible board 315a are arranged on a same surface of the fixation substrate 316, and the second sensor 314b and the second flexible board 315b are arranged on the other surface of the fixation substrate 316. A hardness or rigidity of the fixation substrate 316 is stronger than that of the first flexible board 315 a or the second flexible board 315 b, and provides a support force for the first flexible board 315 a or the second flexible board 315 b. Compared with a conventional hard board, the embodiment of this disclosure can adjust a relative position of the first sensor 314a or a relative position of the second sensor 314b, which is connected with the first flexible board 315a or the second flexible board 315b, by adjusting a position of the first flexible board 315a or the second flexible board 315b, which is more convenient to align the relative positions of the first sensor 314a and the second sensor 314b, facilitates calibration, and improves imaging quality.

In an embodiment, the sensor 314 may be a CSP sensor or a COB sensor, etc. In this disclosure, a COB sensor as shown in FIG. 3 is preferred, because compared with the CSP sensor used in conventional medical endoscopes, the COB sensor is smaller and thinner, and uses gold wire for welding, while the CSP sensor is larger and welded through a bottom welding ball. Using a COB sensor can effectively reduce a volume required for arranging the sensor 314, leave more space for the optical lens group, thereby maximizing the optical lens group and acquiring more image light. A second welding portion 3142 is arranged around the sensor, and a first welding portion which corresponds to the second welding portion 3142 is arranged on the flexible board. The first welding portion and the second welding portion 3142 are welded together, so as to realize electrical connection between the flexible board and the sensor.

The imaging module 31 is an important component of the imaging head for the endoscope to image. The larger the imaging module 31 is, the more light signals the imaging module 31 can receive, thereby presenting a high-quality observation image. However, since the imaging module 31 is arranged inside the long tube 32 and is limited by a diameter of the long tube 32, the space in which the imaging module 31 can be arranged is limited. The embodiments of this disclosure maximize a photosensitive area and ensure an imaging quality by reasonably arranging internal structure(s) for component(s) of the imaging module 31.

In an embodiment, the first sensor 314a, the first flexible board 315a, the fixation substrate 316, the second flexible board 315b and the second sensor 314b are stacked, and positions of these elements can be arranged in a straight line or be stacked in a staggered manner.

In an embodiment, the first flexible board 315a is provided with a through-hole structure, wherein the first sensor 314a is arranged inside said through-hole structure; and/or the second flexible board 315b is provided with a through-hole structure, wherein the second sensor 314b is arranged inside said through-hole structure. A metal plate or a substrate made of other materials may be arranged inside the through-hole structure, so as to provide a support force for the sensor arranged inside the through-hole structure.

In an embodiment, the first prism group 312a includes right-angle prism(s) or triangular prism(s); and/or the second prism group 312b includes right-angle prism(s) or triangular prism(s). A number of prism(s) in the first prism group 312a and the second prism group 312b is not limited, and can be one or more.

In an embodiment, a light-exiting end surface of the first prism group 312a is a rectangle, a long side of said rectangle is parallel to an axial direction of the long tube, and a short side of said rectangle is perpendicular to the axial direction of the long tube; and/or a light-exiting end surface of the second prism group 312b is a rectangle, a long side of said rectangle is parallel to an axial direction of the long tube, and a short side of said rectangle is perpendicular to the axial direction of the long tube. In a special embodiment, the long side is equal to the short side, that is, the light-exiting end surface is a square. In another embodiment, the long side of the light-exiting end surface is longer than the short side. In an embodiment, short side(s) of prism(s) in the first prism group 312a and/or the second prism group 312b is(are) greater than or equal to height(s) of said prism(s), long side(s) of said prism(s) is(are) greater than or equal to short side(s) of said prism(s). In this way, the height of the prism can be fully utilized to maximize the effective image surface, thereby outputting an image of higher resolution. The short side(s) of the prism(s) is(are) less than or equal to the long side(s) of the prism(s), which is also to ensure that the reflective surface of the prism group is fully utilized, so as to output as much image light as possible to achieve a higher resolution output.

In an embodiment, diaphragm(s) may be further added on the light-exiting end surface of the first prism group 312a and/or the second prism group 312b. The diaphragm(s) is(are) arranged on the light-exiting end surface of the first prism group 312a and/or the second prism group 312b, such that the diaphragm(s) block(s) some image lights that cannot be transmitted to the photosensitive area 3141, thereby preventing mutual interference between lights, preventing influence on the accuracy of the sensor 314, and preventing glare, ghosting, etc., which affect the imaging effect.

In an embodiment, a photosensitive area 3141 on a photosensitive surface of the first sensor 314a is greater than or equal to the light-exiting end surface of the first prism group 312a; and/or a photosensitive area 3141 on a photosensitive surface of the second sensor 314b is greater than or equal to the light-exiting end surface of the second prism group 312b. To ensure the imaging effect, the sensor needs to receive as more light signals as possible.

In an embodiment, the light-exiting end surface of the first prism group 312a is arranged on the photosensitive surface of the first sensor 314a, and at least part of the photosensitive area 3141 of the first sensor 314a is in a closed space; and/or the light-exiting end surface of the second prism group 312b is arranged on the photosensitive surface of the second sensor 314b, and at least part of the photosensitive area 3141 of the second sensor 314b is in a closed space, so as to prevent dust and other debris from adhering to or falling into the photosensitive area 3141 of the sensor, thereby ensuring the effectiveness. In another embodiment, a glass plate may also be used and arranged between the first prism group 312a and the first sensor 314a; and/or the glass plate may be arranged between the second prism group 312b and the second sensor 314b. In another embodiment, other polymer transparent plate, such as a plastic plate, etc., may also be used.

As shown in FIG. 2, in an embodiment, the transmission unit 70 includes a signal amplification circuit 33, which is configured to amplify the acquired first electrical signal and/or second electrical signal, and transmit the amplified signal(s) to the processing component for image signal 41, so as to prevent the image signal from being excessively attenuated during the transmission process and thus causing the electrical signal strength received by the processing component for image signal 41 to be too weak to affect the imaging quality.

In an embodiment, a support 313 is further included, and the first optical path component and the second optical path component are arranged at the support 313.

In an embodiment, a 3D electronic endoscope includes an operation portion 40 and an insertion portion 30; wherein the insertion portion 30 and the operation portion 40 may be an integrated structure, and together constitute an imaging head for medical endoscope. Wherein the insertion portion 30 includes a long tube 32, an imaging module 31, an illumination optical path and a transmission unit 70; wherein the long tube 32 is a tubular structure with a hollow interior, and the imaging module 31 is arranged inside the long tube 32. The illumination optical path and the transmission unit 70 are arranged inside the long tube 32; the illumination optical path is configured to illuminate a designated region of an inspected object 100 with a light from a light source 10.

The imaging module 31 is configured to receive an image light which is reflected or excited by a designated region and obtained by a distal end of the insertion portion 30, convert the image light into an electrical signal, and transmit the electrical signal to the operation portion 40 through the transmission unit 70.

The operation portion 40 includes an operation handle housing, a controller and a processing component for image signal 41; wherein the controller is configured to implement different functions according to signal(s), which is(are) inputted by an operator; the processing component for image signal is configured to process the electrical signal which is transmitted by the transmission unit 70, so as to obtain an image signal.

The imaging module 31 includes an optical path component, a sensor, a flexible board, and a fixation substrate 316.

The optical path component includes an objective lens group and a prism group; wherein the objective lens group is configured to receive the image light which is reflected or excited by the designated region; the prism group is configured to reflect or transmit the image light to the sensor; the sensor is electrically connected with the flexible board, and configured to convert the image light into the electrical signal.

The flexible board is electrically connected with the transmission unit 70, so as to transmit the electrical signal to the transmission unit 70; one sensor is respectively arranged on one respective side of the fixation substrate 316, wherein the sensor and the flexible board are arranged on a same surface of the fixation substrate 316. A hardness or rigidity of the fixation substrate 316 is greater than that of the flexible board, so as to provide a support force for the flexible board.

The above specific examples are used to illustrate this disclosure, which are only used to help understand this disclosure and are not intended to limit this disclosure.

## Claims

1. A 3D electronic endoscope, **characterized in that**, comprising an insertion portion and an operation portion;
wherein the insertion portion comprises a long tube, an imaging module, an illumination optical path, and a transmission unit; the long tube is a tubular structure with a hollow interior, the imaging module is arranged inside the long tube, the illumination optical path and the transmission unit are also arranged inside the long tube; the illumination optical path is configured to illuminate a designated region of an inspected object with a light; the imaging module is configured to receive an image light which is reflected or excited by the designated region and obtained by a distal end of the insertion portion, convert the image light into an electrical signal, and transmit the electrical signal to the operation portion through the transmission unit;
wherein the operation portion comprises an operation handle housing, a controller, and a processing component for image signal; the controller and the processing component for image signal are arranged inside the operation handle housing; the controller is configured to implement different functions according to signal(s) which is(are) inputted by an operator; the processing component for image signal is configured to process the electrical signal which is transmitted by the transmission unit, so as to obtain an image signal;
wherein the imaging module comprises a first optical path component, a second optical path component, a first sensor, a second sensor, a first flexible board, a second flexible board, and a fixation substrate; the first optical path component comprises a first objective lens group and a first prism group, the second optical path component comprises a second objective lens group and a second prism group; the first objective lens group is configured to receive the image light along a first path which is reflected or excited by the designated region, the first prism group is configured to reflect or transmit the image light along the first path to the first sensor; the first sensor is electrically connected with the first flexible board, and configured to convert the image light along the first path into a first electrical signal; the second objective lens group is configured to receive the image light along a second path which is reflected or excited by the designated region; the second prism group is configured to reflect or transmit the image light along the second path to the second sensor; the second sensor is electrically connected with the second flexible board, and configured to convert the image light along the second path into a second electrical signal;
the first flexible board and the second flexible board are electrically connected with the transmission unit, so as to transmit the first electrical signal and the second electrical signal to the transmission unit;
the first sensor and the second sensor are arranged on respective sides of the fixation substrate; the first sensor and the first flexible board are arranged on one surface of the fixation substrate; the second sensor and the second flexible board are arranged on the other surface of the fixation substrate.

2. The 3D electronic endoscope according to claim 1, **characterized in that**, the first sensor, the first flexible board, the fixation substrate, the second flexible board and the second sensor are stacked.

3. The 3D electronic endoscope according to claim 1, **characterized in that**, the first flexible board is provided with a through-hole structure, wherein the first sensor is arranged inside said through-hole structure; and/or
the second flexible board is provided with a through-hole structure, wherein the second sensor is arranged inside said through-hole structure.

4. The 3D electronic endoscope according to claim 1, **characterized in that**, the first prism group comprises right-angle prism(s) or triangular prism(s); and/or
the second prism group comprises right-angle prism(s) or triangular prism(s).

5. The 3D electronic endoscope according to claim 1, **characterized in that**, a light-exiting end surface of the first prism group is a rectangle; wherein a long side of said rectangle is parallel to an axial direction of the long tube, and a short side of said rectangle is perpendicular to the axial direction of the long tube; and/or
a light-exiting end surface of the second prism group is a rectangle; wherein a long side of said rectangle is parallel to an axial direction of the long tube, and a short side of said rectangle is perpendicular to the axial direction of the long tube.

6. The 3D electronic endoscope according to claim 1, **characterized in that**, further comprising diaphragm(s), which is(are) arranged on a light-exiting end surface of the first prism group and/or a light-exiting end surface of the second prism group.

7. The 3D electronic endoscope according to claim 1, **characterized in that**, a photosensitive area on a photosensitive surface of the first sensor is greater than or equal to a light-exiting end surface of the first prism group; and/or
a photosensitive area on a photosensitive surface of the second sensor is greater than or equal to a light-exiting end surface of the second prism group.

8. The 3D electronic endoscope according to claim 7, **characterized in that**, the light-exiting end surface of the first prism group is arranged on the photosensitive surface of the first sensor, and at least part of the photosensitive area on the photosensitive surface of the first sensor is in a closed space; and/or
the light-exiting end surface of the second prism group is arranged on the photosensitive surface of the second sensor, and at least part of the photosensitive area on the photosensitive surface of the second sensor is in a closed space.

9. The 3D electronic endoscope according to claim 1, **characterized in that**, further comprising glass plate(s), which is(are) arranged between the first prism group and the first sensor, and/or between the second prism group and the second sensor.

10. The 3D electronic endoscope according to claim 1, **characterized in that**, the transmission unit comprises a signal amplification circuit, which is configured to amplify the first electrical signal and/or the second electrical signal, and transmit the amplified signal(s) to the processing component for image signal.

11. The 3D electronic endoscope according to claim 1, **characterized in that**, the fixation substrate comprises a first fixation substrate and a second fixation substrate; wherein the first sensor and the first flexible board are arranged on the first fixation substrate; the second sensor and the second flexible board are arranged on the second fixation substrate.

12. The 3D electronic endoscope according to any one of claims 1-11, **characterized in that**, the fixation substrate is a heat conduction plate for conducting heat which is generated by the sensor(s).

13. The 3D electronic endoscope according to any one of claims 1-12, **characterized in that**, the fixation substrate is a ceramic plate or a metal plate.

14. The 3D electronic endoscope according to claim 1, **characterized in that**, further comprising a support; wherein the first optical path component and the second optical path component are arranged at the support.

15. A 3D electronic endoscope assembly, **characterized in that**, comprising an insertion portion and an operation portion;
wherein the insertion portion comprises a long tube, an imaging module, an illumination optical path, and a transmission unit; the long tube is a tubular structure with a hollow interior, the imaging module is arranged inside the long tube, the illumination optical path and the transmission unit are also arranged inside the long tube; the illumination optical path is configured to illuminate a designated region of an inspected object with a light; the imaging module is configured to receive an image light which is reflected or excited by the designated region and obtained by a distal end of the insertion portion, convert the image light into an electrical signal, and transmit the electrical signal to the operation portion through the transmission unit;
wherein the operation portion comprises an operation handle housing, a controller, and a processing component for image signal; the controller and the processing component for image signal are arranged inside the operation handle housing; the controller is configured to implement different functions according to signal(s) which is(are) inputted by an operator; the processing component for image signal is configured to process the electrical signal which is transmitted by the transmission unit, so as to obtain an image signal;
wherein the imaging module comprises an objective lens group, a prism group, a sensor, a fixation substrate, and a flexible board; wherein the objective lens group is configured to receive the image light which is reflected or excited by the designated region; the prism group is configured to reflect or transmit to the sensor the image light which is received by the objective lens group; wherein the sensor is electrically connected with the flexible board, and configured to convert the image light into the electrical signal; the flexible board is electrically connected with the transmission unit, and configured to transmit the electrical signal to the transmission unit;
the sensor and the flexible board are electrically connected with each other, wherein the sensor and the flexible board are arranged on the fixation substrate.

16. An imaging system for 3D electronic endoscope, **characterized in that**, comprising a light source, a light guide beam, an imaging host, a cable, and the 3D electronic endoscope according to any one of claims 1-14; wherein the light source is connected with the 3D electronic endoscope through the light guide beam, and one end of the 3D electronic endoscope is connected with the imaging host through the cable.
